# EUROPEAN PATENT APPLICATION

(11) **EP 3 627 516 A1**
(43) Date of publication of application: **25.03.2020**
(21) Application number: 19195840.4
(22) Date of filing: 06.09.2019
(51) Int. Cl.: G16H 40/20, G16H 40/40

(54) **SYSTEM AND METHOD FOR CAREGIVER SHIFT CHANGE**

(30) Priority: 19.09.2018 US 201862733449 P
(71) Applicant: Hill-Rom Services, Inc., Batesville, IN 47006-9167 (US)
(72) Inventor: ROBINSON, Andrew S, Batesville, IN Indiana 47006-9167 (US); BAKER, Steven D, Batesville, IN Indiana 47006-9167 (US); SMITH, Bradley T, Batesville, IN Indiana 47006-9167 (US); AGDEPPA, Eric D, Batesville, IN Indiana 47006-9167 (US); WELLS, Pamela, Batesville, IN Indiana 47006-9167 (US); HASSEY, Laura A, Batesville, IN Indiana 47006-9167 (US); MYERS, Thomas A, Batesville, IN Indiana 47006-9167 (US)
(74) Representative: Findlay, Alice Rosemary

(57) **Abstract**

A caregiver shift change system and method for a healthcare facility includes a patient bed in a patient room and a high-accuracy locating system that includes locating tags. A first tag is coupled to a first caregiver and a second tag is coupled to a second caregiver. The high-accuracy locating system further has a number of transceivers within the healthcare facility to receive tag data from the tags. The high-accuracy locating system also includes a computer coupled to the transceivers to receive the tag data and to process the tag data to determine a location of each tag within the healthcare facility. The caregiver shift change system and method also includes a server that determines whether a successful caregiver shift change has occurred based on locations of the first and second tags being, for a predetermined period of time, within a threshold proximity of each other and within a zone defined around the patient support apparatus in the patient room.

## Description

The present disclosure relates to systems and methods of caregiver shift changes and particularly, to patient handoffs from one caregiver to another during caregiver shift changes. More particularly, the present disclosure relates to ensuring that outgoing and incoming caregivers adequately discuss all patients during the caregiver shift changes.

In healthcare facilities, outgoing caregivers at the end of their shifts typically provided information to incoming caregivers regarding the patients to whom they have been assigned. This is sometimes referred to as a patient handoff. During patient handoffs between caregivers, it is desired that the outgoing caregivers accurately communicate relevant information about all of their assigned patients to corresponding incoming caregivers to enhance patient care. The complexity and nuance of the type of information, communication methods, and various caregivers has a bearing on the effectiveness and efficiency of the handoff as well as on patient care. Thus, there is an ongoing need for improvements in systems and methods for caregiver shift changes.

An apparatus, system, or method may comprise one or more of the following features alone or in any combination.

According to a first aspect of the present disclosure, a caregiver shift change system for use in a healthcare facility having a plurality of patient rooms may be provided. The caregiver shift change system may include a patient support apparatus in a first patient room of the plurality of patient rooms and a high-accuracy locating system that may include a plurality of locating tags. A first tag of the plurality of tags may be coupled to a first caregiver, a second tag of the plurality of tags may be coupled to a second caregiver. The high-accuracy locating system further may have a plurality of transceivers that may be mounted within the healthcare facility and that may be configured to receive tag data from the plurality of tags. The high-accuracy locating system also may include at least one computer that may be coupled to the plurality of transceivers to receive the tag data therefrom and that may process the tag data to determine a location of each tag of the plurality of tags within the healthcare facility. The caregiver shift change system also may include a server that may be configured to determine whether a successful caregiver shift change may have occurred based on locations of the first and second tags being, for a predetermined period of time, within a threshold proximity of each other and within a zone defined around the patient support apparatus in the first patient room.

In some embodiments, the high- accuracy locating system may operate according to an ultra-wideband (UWB) technology. For example, the at least one computer may use two way ranging and time difference of arrival data (TDOA) techniques to determine the location of each tag of the plurality of tags.

It is contemplated by the present disclosure that the at least one computer may use the tag data from only a subset of the plurality of transceivers to determine the location of each tag of the plurality of tags. The subset may be determined based on signal strength of signals that may include the tag data and that may be communicated between each tag of the plurality of tags and one or more transceivers of the plurality of transceivers. For example, the subset may include at least three transceivers from the plurality of transceivers that may have highest signal strength values as compared to others of the plurality of transceivers.

If desired, the threshold proximity may be about three feet. Alternatively or additionally, the zone defined around the patient support apparatus may be defined as an area within about three feet of the patient support apparatus. Further alternatively or additionally, the zone defined around the patient support apparatus may be defined as an area within about six feet of a third tag of the plurality of tags mounted to the patient support apparatus. Optionally, the zone defined around the patient support apparatus may be defined by a set of X and Y coordinates within the first patient room as mapped within the at least one computer. Further optionally, the zone defined around the patient support apparatus may be defined as any area within the first patient room that may be beyond a threshold distance from a doorway of the first patient room.

In some embodiments, the patient support apparatus may include a sensor that may sense a presence of a patient on the patient support apparatus and the server may be configured to determine whether a successful caregiver shift change has occurred only when the patient is present on the patient support apparatus as sensed by the sensor. Therefore, the patient support apparatus may include communication circuitry that may be configured to transmit sensor data from the sensor for receipt by the server. If desired, the sensor may include a weight sensor of a weigh scale system of the patient support apparatus.

According to a second aspect of the present disclosure, a method of detecting a successful caregiver shift change in a healthcare facility having patient rooms may be provided. The method may include receiving, at a computer of a high-accuracy locating system, tag data from a plurality of tags of the high-accuracy locating system. A first tag of the plurality of tags may be coupled to a first caregiver and a second tag of the plurality of tags may be coupled to a second caregiver. The high-accuracy locating system further may have a plurality of transceivers mounted within the healthcare facility and that may be configured to receive tag data from the plurality of tags. The computer may be coupled to the plurality of transceivers to receive the tag data therefrom. The method further may include determining, at the computer, a location of the each tag of the plurality of tags and determining, at the computer, whether a successful caregiver shift change has occurred based on locations of the first and second tags being, for a predetermined period of time, within a threshold proximity of each other and within a zone defined in a first patient room adjacent a patient support apparatus.

In some embodiments, determining, at the computer, the location of each tag may include determining the location of the each tag using an ultra-wideband (UWB) technology. For example, determining, at the computer, the location of each tag may include using two way ranging and time difference of arrival (TDOA) techniques.

It is contemplated by the present disclosure that determining, at the computer, the location of each tag may include using the tag data from only a subset of the plurality of transceivers to determine the location of each tag of the plurality of tags. The subset may be determined based on signal strength of signals that may include the tag data and that may be communicated between each tag of the plurality of tags and one or more transceivers of the plurality of transceivers. For example, the subset may include at least three transceivers from the plurality of transceivers that may have highest signal strength values as compared to others of the plurality of transceivers.

If desired, the threshold proximity in the method may be about three feet. Alternatively or additionally, the zone defined adjacent the patient support apparatus in the method may be defined as an area within about three feet of the patient support apparatus. Further alternatively or additionally, the zone defined adjacent the patient support apparatus in the method may be defined as an area within about six feet of a third tag of the plurality of tags that may be mounted to the patient support apparatus. Optionally, the zone defined adjacent the patient support apparatus in the method may be defined by a set of X and Y coordinates within the first patient room as mapped within the computer. Further optionally, the zone defined adjacent the patient support apparatus may be defined as any area within the first patient room that may be beyond a threshold distance from a doorway of the first patient room.

In some embodiments of the method, the patient support apparatus may include a sensor that may sense a presence of a patient on the patient support apparatus and determining, at the computer, whether a successful caregiver shift change has occurred may be undertaken only when the patient is present on the patient support apparatus as sensed by the sensor. Therefore, the patient support apparatus of the method may include communication circuitry that may be configured to transmit sensor data from the sensor for receipt by the computer. For example, the sensor of the method may include a weight sensor of a weigh scale system of the patient support apparatus.

According to a third aspect of the present disclosure, a caregiver shift change system for use in a healthcare facility having a plurality of patient rooms may be provided. The caregiver shift change system may include a high-accuracy locating system including a plurality of locating tags. A first tag of the plurality of tags may be coupled to a first caregiver, a second tag of the plurality of tags may be coupled to a second caregiver, and a third tag of the plurality of tags may be coupled to a patient. The high-accuracy locating system further may have a plurality of transceivers that may be mounted within the healthcare facility and that may be configured to receive tag data from the plurality of tags. At least one computer may be coupled to the plurality of transceivers to receive the tag data therefrom and process the tag data to determine a location of each tag of the plurality of tags within the healthcare facility. A server may be configured to determine whether a successful caregiver shift change has occurred based on locations of the first and second tags being, for a predetermined period of time, within a threshold proximity of each other and within a zone defined around the third tag in a patient room of the healthcare facility.

In some embodiments, the high- accuracy locating system of the third aspect may operate according to an ultra-wideband (UWB) technology. If desired, the at least one computer of the third aspect may use two way ranging and time difference of arrival data (TDOA) techniques to determine the location of each tag of the plurality of tags. It is contemplated that the at least one computer of the third aspect may use the tag data from only a subset of the plurality of transceivers to determine the location of each tag of the plurality of tags. The subset may be determined based on signal strength of signals that may include the tag data and that may be communicated between each tag of the plurality of tags and one or more transceivers of the plurality of transceivers. The subset may include, for example, at least three transceivers from the plurality of transceivers that may have highest signal strength values as compared to others of the plurality of transceivers.

Optionally, the threshold proximity of the third aspect may be about three feet. Further optionally, the zone defined around the third tag may be defined as an area within about three feet of the third tag. Alternatively, the zone defined around the third tag may be defined as an area within about six feet of the third tag.

According to a fourth aspect of the present disclosure, a method of detecting a successful caregiver shift change in a healthcare facility having patient rooms may be provided. The method may include receiving, at a computer of a high-accuracy locating system, tag data from a plurality of tags of the high-accuracy locating system. A first tag of the plurality of tags may be coupled to a first caregiver, a second tag of the plurality of tags may be coupled to a second caregiver, and a third tag of the plurality of tags may be coupled to a patient. The high-accuracy locating system further may have a plurality of transceivers that may be mounted within the healthcare facility and that may be configured to receive tag data from the plurality of tags. The computer may be coupled to the plurality of transceivers to receive the tag data therefrom. The method further may include determining, at the computer, a location of the each tag of the plurality of tags and determining, at the computer, whether a successful caregiver shift change has occurred based on locations of the first and second tags being, for a predetermined period of time, within a threshold proximity of each other and within a zone defined in a first patient room around the third tag.

In some embodiments of the fourth aspect, determining, at the computer, the location of each tag may include determining the location of the each tag using an ultra-wideband (UWB) technology. Optionally, therefore, determining, at the computer, the location of each tag may include using two way ranging and time difference of arrival (TDOA) techniques. It is further contemplated that determining, at the computer, the location of each tag may include using the tag data from only a subset of the plurality of transceivers to determine the location of each tag of the plurality of tags. For example, the subset may be determined based on signal strength of signals that may include the tag data and that may be communicated between each tag of the plurality of tags and one or more transceivers of the plurality of transceivers. The subset may include at least three transceivers from the plurality of transceivers that may have highest signal strength values as compared to others of the plurality of transceivers.

Optionally, the threshold proximity of the fourth aspect may be about three feet. Alternatively or additionally, the zone defined around the third tag may be defined as an area within about three feet of the third tag. Alternatively, the zone defined around the third tag may be defined as an area within about six feet of the third tag.

The invention will now be further described by way of example with reference to the accompanying drawings, in which:
Fig. 1 is diagrammatic view of a caregiver shift change system showing a patient support apparatus on which a patient is supported and a high-accuracy real time locating system (RTLS) including a plurality of tags, a plurality of ultra-wideband (UWB) locating transceivers wirelessly communicating with the plurality of tags, a UWB hub computer in communication with the UWB transceivers, and an RTLS server in communication with UWB hub computer via a network;
Fig. 2 is a block diagram showing electrical circuitry of the patient support apparatus and showing an equipment locating tag of the plurality of tags in communication with one or more UWB transceivers of the high-accuracy RTLS; and
Figs. 3 and 4 together form a flow diagram of at least one embodiment of a method for determining successful handoffs of all patients of an outgoing caregiver to an incoming caregiver during a shift change.

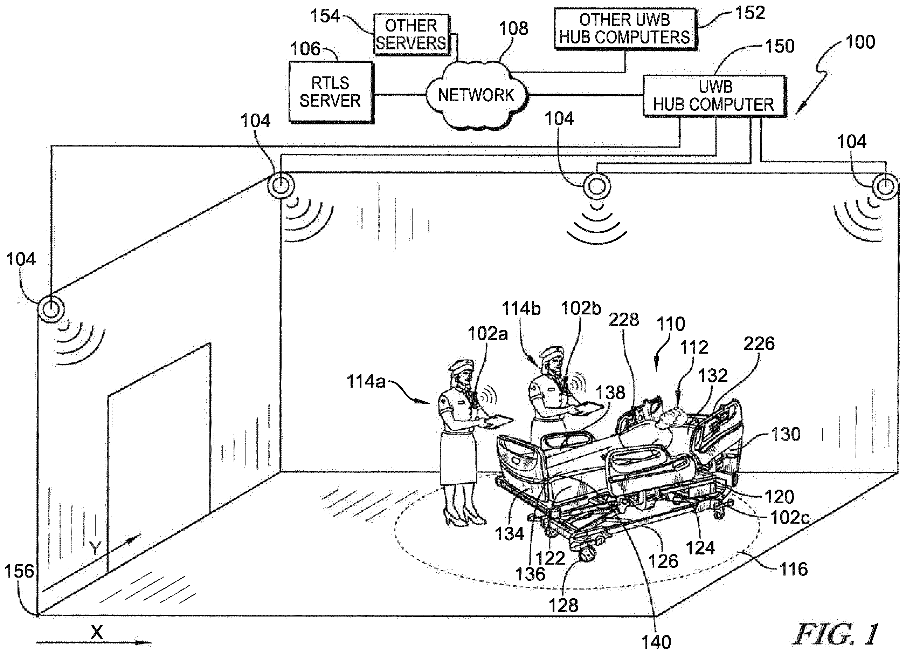

A caregiver shift change system 100 of a healthcare facility is configured to determine successful handoffs of one or more patients from an outgoing caregiver 114b to an incoming caregiver 114a. In some embodiments, a successful shift change occurs based on system 100 determining proximity of at least two caregivers 114a, 114b (e.g., incoming and outgoing caregivers, respectively), for a predetermined amount of time, within a zone 116 adjacent to a patient support apparatus 110 that is configured to support a patient 112 as shown in Fig. 1. Thus, if an outgoing caregiver 114b is assigned to a single patient 112, then a successful caregiver shift change occurs based on the single patient 112 being handed off to the incoming caregiver 114a in the manner just described. If the outgoing caregiver 114b is assigned to multiple patients 112, then a successful caregiver shift change occurs based on each of the multiple patients 112 being handed off to one or more incoming caregivers 114a in the manner just described. In some instances, the outgoing caregiver 114b hands off all assigned patients 112 to the same incoming caregiver 114a, but this need not be the case. That is, the outgoing caregiver 114b may handoff the assigned patients 112 to different incoming caregivers 114a according to some scenarios contemplated herein. Similarly, an incoming caregiver 114a may have patients 112 handed off from multiple outgoing caregivers 114b. In each of these situations, system 100 tracks the patient handoffs between caregivers 114a, 114b to determine successful caregiver shift changes.

The illustrated patient support apparatus 110 is embodied as a patient bed 110. However, it should be appreciated that this disclosure is applicable to other types of patient support apparatuses, including other types of beds, surgical tables, examination tables, stretchers, chairs, wheelchairs, patient lifts and the like. In the description below, patient support apparatus 110 is sometimes referred to as patient bed 110 or just bed 110. However, the description is equally applicable to other types of patient support apparatus 110 in a healthcare facility.

The overall system 100 is subdivided into sub-systems which are themselves, also referred to herein as "systems." For example, system 100 includes a locating system, sometimes referred to as a real time locating system (RTLS) in the art, that tracks the locations of caregivers and equipment throughout the healthcare facility. In some embodiments, the locating system is embodied as a high-accuracy locating system such as an ultra-wideband (UWB) locating system, but this need not be the case in other embodiments of high-accuracy locating systems such as those using radio detection and ranging (RADAR) equipment or cameras and/or other imaging equipment. The illustrative locating system includes a plurality of transceivers 104 positioned throughout the healthcare facility such as in the patient room of Fig. 1, in the hallway of the healthcare facility, and in other locations throughout the healthcare facility (e.g, staff break rooms, bathrooms, pharmacy, treatment rooms, operating rooms, imaging rooms, laboratories, cafeteria, etc.) at the discretion of the system designer.

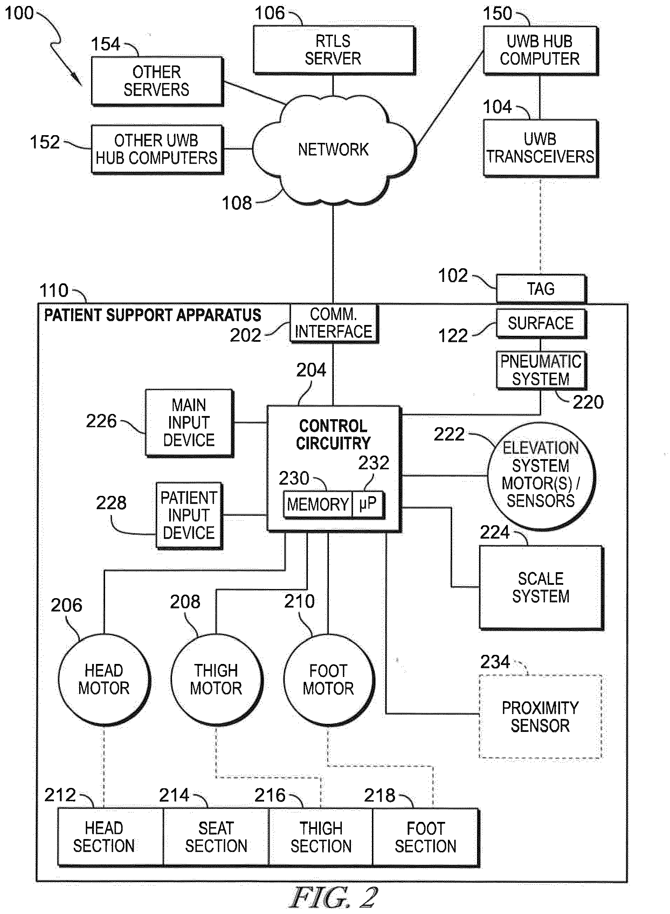

The transceivers 104 receive wireless transmissions from caregiver locating tags 102a, 102b that are worn by respective caregivers 114a, 114b and from equipment tags 120c that are attached to various pieces of equipment such as patient beds 110. Tags 102a, 102b, 102c are sometimes just referred to herein as tags 102 and such a generic tag 120 is shown diagrammatically in Fig. 2 attached to patient support apparatus 110. In the example of Fig. 1, tags 102a, 102b are coupled to the clothing of caregivers 114a, 114b, such as with a clip, and tag 102c is attached to the patient bed 110 such as with a fastener (e.g., bolt, screw, snap, hook-and-loop fastener, adhesive, magnet, etc.). Tags 102a, 102b may instead be worn around the caregivers' necks on a necklace or attached to the caregivers' wrists on a wristband or bracelet, for example.

In some embodiments, the tags 102 receive a signal from the transmitter circuitry of one or more of the transceivers 104 and, in response, transmit a return signal to at least one of the transceivers 104. The return signal includes a tag identification (ID) which is unique to each tag 102. Such an arrangement preserves battery life of tags 102 because transmissions of tag ID's are only made by the tags 102 when in communicative proximity of one or more transceivers 104 and after receiving a request signal from at least one of the transceivers 104. In other embodiments, tags 102 transmit their respective tag ID's on a periodic basis. In still other embodiments, short range wireless beacons or infrared transmitters are mounted at fixed locations throughout the healthcare facility and send a signal with a location ID to the tags 102 that are in the vicinity of the short range beacons and, in response to receipt of the signal, the tags 102 transmit their respective tag ID's and the location ID's to transceivers 104. In each of these embodiments, transceivers 104 transmit the received tag ID or tag ID's to an RTLS server 106 of the locating system along with a respective transceiver ID and, if applicable, the location ID.

In some embodiments, the transceiver ID's correlate to particular locations in the healthcare facility. Thus, the RTLS server 106 determines the locations of tags 102 within the healthcare facility by correlating the tag ID's with the receiver ID's (and/or the location ID's, if applicable) and, ultimately, with the location correlated with the receiver ID's and/or location ID's. RTLS server 106 also correlates the tag ID's with the respective caregivers wearing tags 102 and with the equipment to which tags 102 are attached. In some embodiments, patients 112 also have tags 102 for tracking the whereabouts of the patients 112 throughout the healthcare facility. Thus, in some embodiments, the locating system of overall system 100 includes tags 102, transceivers 104, and RTLS server 106. Tags 102 are sometimes referred to as "badges" and so the terms "tag" and "badge" are used interchangeably herein.

System 100 includes network infrastructure which is designated diagrammatically as network 108 in Figs. 1 and 2. Network 108 is intended to represent the infrastructure (e.g., wireless access points, Ethernet jacks such as RJ-45 connectors, wires, routers, gateways, etc.) provided in a healthcare facility and the various computer devices (e.g., personal computers, servers, laptop computers, patient care equipment, etc.) that are coupled to the infrastructure. The various subsystems described herein include components that may communicate with each other using portions of network 108. In the illustrative example, transceivers 104 communicate with RTLS server 106 via portions of network 108.

In some embodiments, tags 102 communicate wirelessly with transceivers 104 using infrared (IR) technology. In such embodiments, line of sight between tags 102 and one or more of transceivers 104 needs to remain unobstructed in order for communication to be established between the tags 102 and one or more of the transceivers 104 to determine the location of the tags 102 in the healthcare facility. Thus, the IR signals cannot pass through walls, equipment, and people located in the room. In general, locating systems that use IR communication between tags 102 and transceivers 104 are able to reliably determine that the tags 102 are located inside a particular room, but are not able to determine the exact location, within a relatively small accuracy threshold, of the tag 102 within the room.

As noted above, the locating system in some embodiments is embodied as a high-accuracy locating system such as an ultra-wideband (UWB) locating system. In such embodiments, tags 102 are configured as UWB tags 102 having UWB transceivers, and transceivers 104 are configured as UWB transceivers. The UWB transceivers 104 are stationary and the UWB transceivers of tags 102 are mobile, but their circuitry otherwise may be substantially the same. Thus, tags 102 and transceivers 104 each include a housing that contains associated circuitry. The circuitry of tags 102 and transceivers 104 includes, for example, a processor such as a microprocessor or microcontroller or the like, memory for storing software, and communications circuitry including a transmitter, a receiver and at least one antenna. Transceivers 104 each include mounting hardware, such as brackets or plates or the like, in some embodiments, to permit the transceivers 104 to be mounted at fixed locations in the patient rooms and other locations of the healthcare facility with fasteners such as screws or the like.

In the illustrative example of system 100 of Fig. 1, the high-accuracy locating system further includes an UWB hub computer 150 which is communicatively coupled to other UWB hub computers 152 of the high-accuracy locating system via network 108 of the healthcare facility. UWB hub computer 150 serves as an intermediary between transceivers 104 and RTLS server 106. Of course, the other UWB hub computers 152 are also communicatively coupled to respective sets of transceivers 104. In the illustrative example, the high-accuracy locating system is also communicatively coupled to other servers or computers 154 of the healthcare facility, such as to a nurse call server, an EMR server, or an admission/ discharge/transfer (ADT) computer, just to name a few. The other servers and computers 154 block in Figs. 1 and 2, therefore, generically represents all other computers and servers of network 108 in a healthcare facility.

As shown diagrammatically in Fig. 1, various lines interconnect transceivers 104 with hub computer 150 and interconnect servers and computers 106, 152, 154 with each other via network 108. It should be appreciated that these lines represent bidirectional communication over wired data links (including electrical wires or fiber optic data links) and/or wireless data links, at the discretion of the designer of system 100. UWB transceivers 104 communicate wirelessly with tags 102 using radio frequency (RF). It is known that RF signals are able to pass through walls, ceilings, floors, and other objects such as people and equipment. Thus, according to this disclosure, it is not required that each patient room has a transceiver 104 located therein in those embodiments of the locating system using RF communication.

According to this disclosure, the portion of system 100 that operates as a high-accuracy locating system using UWB technology is able to determine the location of each tag 102 that is in communication with at least three of transceivers 104 within about one foot (30.48 cm) or less of the tag's actual location. In other embodiments, the locating system is able to determine the location of each tag 102 that is in communication with at least three of transceivers 104 within about three feet (91.44 cm) or less of the tag's actual location and such embodiments are still considered to be high-accuracy locating systems according to the present disclosure.

In some embodiments, the high-accuracy locating system is operable to determine the location of tags 102 in 3-dimensional space. However, in many embodiments, it suffices to determine the location of tags 102 in 2-dimensional space. Accordingly, Fig. 1 shows X and Y directions relative to a floor plan of the healthcare facility with point 156 serving as an arbitrary origin of an X-Y coordinate system. The Z dimension corresponds to a height in a Z direction (not shown) above the floor plan of Fig. 1. UWB locating systems typically operate within the 3.1 gigahertz (GHz) to 10.6 GHz frequency range. Suitable transceivers 104 in this regard include WISER Mesh Antenna Nodes and suitable tags 102 in this regard include Mini tracker tags, all of which are available from Wiser Systems, Inc. of Raleigh, North Carolina and marketed as the WISER LOCATOR™ system.

In some embodiments, the high-accuracy locating system implementing UWB technology uses 2-way ranging, clock synchronization, and time difference of arrival (TDOA) techniques to determine the locations of tags 102 in the X and Y directions (and, optionally, the Z direction in some embodiments). See, for example, International Publication No. WO 2017/083353 A1, which is hereby incorporated by reference herein in its entirety for all that it teaches to the extent not inconsistent with the present disclosure which shall control as to any inconsistencies, for a detailed discussion of the use of these techniques in a UWB locating system. Using these techniques, distances between the stationary transceivers 104 and the various mobile tags 102 are determined based on bidirectional wireless signals communicated between tags 102 and transceivers 104. For example, the distance from each transceiver 104 to any particular tag 102 can be resolved onto the X-Y plane as a circle having a radius equal to the distance and having its center at the particular transceiver 104. The actual location of the mobile tag 102 is determined based on the point of intersection of three or more of the circles defined by radii from three or more corresponding transceivers 104.

The location of each stationary transceiver 104 is mapped onto the X-Y coordinate system by server 106. Thus, each transceiver has its own X and Y coordinates relative to origin 156. As the mobile tags 102 move throughout the healthcare facility, server 106 determines the X and Y coordinates of the various mobile tags 102 relative to origin 156 based on the distances from the known X and Y coordinates of the transceivers 104.

It should be appreciated that, unless a tag 102 is midway between two transceivers 104 on a straight line connecting the two transceivers 104 (in which case the two circles generated will be tangent to each other at a single point), then two circles that are generated from the two transceivers 104 will intersect at two points such that a circle generated from a third transceiver 104 is needed to determine which of the two points is the one corresponding to the location of the tag 102. Generating fourth, fifth, sixth, etc. circles having other transceivers 104 as their respective centers will further increase the accuracy of determining the actual location of the particular tag 102. Due to small errors introduced by refraction of the RF signal through solid objects, including walls, people, equipment, etc., the three or more circles in many instances will not intersect at exactly the same point and so interpolation between clusters of circle intersections is performed to arrive at the calculated location of the particular mobile tag 102 of interest on the X-Y plane. These considerations are discussed in International Publication No. WO 2017/083353 A1 which is already incorporated by reference herein.

Tracking the locations of multiple mobile tags 102 in substantially real time using 2-way ranging, clock synchronization, TDOA, resolution of circles onto the X-Y plane, and interpolating intersection point clusters of the circles requires a large amount of computational power by hub computers 150, 152 and/or the associated RTLS server 106. Thus, each hub computer 150, 152 of the high-accuracy locating system receives incoming data from a predetermined number of transceivers 104. In the illustrative example of Fig. 1, hub computer 150 receives data from four transceivers 104. TDC Acquisition Holdings, Inc. of Huntsville, Alabama which does business as Time Domain, makes a hub computer (referred to as the PLUS Synchronization Distribution Panel) that is capable of receiving incoming data from up to 144 transceivers. The locating server or computer 106, in turn, receives data from the various hubs 150, 152 and tracks or monitors the locations of tags 102 in the healthcare facility.

Regardless of the number of transceivers 104 coupled to hub computers 150, 152, it is contemplated by the present disclosure that, in some embodiments, locating server 106 and/or hub computers 150, 152 are programmed to use signals from only a subset of the plurality of transceivers 104 to determine the location of any given locating tag 102. For example, the subset may be determined based on signal strength of signals between the particular locating tag 102 and the plurality of transceivers 104. The subset may include at least three transceivers 104 from the plurality of transceivers 104 having highest signal strength values as compared to others of the plurality of transceivers 104.

The caregiver shift change system 100 shown in Fig. 1 includes locating tags 102a, 102b worn by caregivers 114a, 114b and locating tag 102c mounted to patient support apparatus 110. Transceivers 104 are configured to receive wireless signals from the tags 102a, 102b, 102c and the computer 150 and/or server 106 determines locations of the respective caregivers 114a, 114b and the patient support apparatus 110 with high-accuracy. Thus, the locations of tags 102a, 102b, 102c are considered to be the locations of the respective caregivers 114a, 114b and patient support apparatus 110. That is, server 106 determines the X and Y coordinates of each of tags 102a, 102b, 102c relative to origin 156. In some embodiments, the high-accuracy locating system portion of caregiver shift change system 100 determines a location of each tag 102a, 102b, 102c within about three feet or less, such as about one foot, of the actual location of the respective tag 102a, 102b, 102c.

As mentioned above, a successful shift change occurs based on system 100 determining proximity of at least two caregivers 114a, 114b (e.g., incoming and outgoing caregivers, respectively), for a predetermined amount of time, within zone 116 adjacent to a patient support apparatus 110 as shown in Fig. 1. Thus, zone 116 is delineated by a set of points having X and Y coordinates that are stored in one or more computer devices of system 100 (e.g., stored within server 106) or is otherwise modeled mathematically or is superimposed on a model of a floor plan of the healthcare facility.

In some embodiments, it is server 106 that is configured with software which makes the determination regarding successful shift changes based on patient handoffs, but in other embodiments, one of the other servers or computers 154, such as a workflow server, nurse call server, scheduling server, etc. is configured with the software that makes the determination regarding successful shift changes based on patient handoffs. Accordingly, the present disclosure describes server 106 as performing various calculations and functions to determine whether a successful shift change has occurred but the discussion is equally applicable to other computers, such as computers 150, 152, 154. That is some functions described herein as being performed by server 106 may, in some embodiments, be distributed among multiple computer devices 106, 150, 152, 154.

In some embodiments, zone 116 around patient support apparatus 100 is defined as an area within about three feet of the patient support apparatus 110. That is zone 116 is modeled as an area three feet beyond a perimeter of a footprint of the patient bed. Zone 116, therefore, may be defined as a geometric footprint, such as a rectangle, as measured with respect to tag 102c that is attached to the patient support apparatus 116. Illustratively, the geometric footprint is a circle that is about six feet in radius from tag 102c. Thus, zone 116 is defined as an area within about six feet of tag 102c mounted to the patient support apparatus 110. Assuming tag 102c is mounted along a centerline of bed 110, then zone 116 will extend about four feet beyond bed 110 in some areas assuming a width of bed 110 is about four feet.

It is known that some patient beds 110 are placed at particular locations within patient rooms. For example, a head wall unit or bed locator unit may be mounted to a wall in a patient room and the patient bed may be placed with its head end centered on the head wall unit or bed locator unit. See, for example, U.S. Patent No. 6,145,253 for examples of such head wall units and bed locator units. If patient bed 110 is expected to be situated at a particular location within a patient room, then in such embodiments, zone 116 may be defined around the patient support apparatus 110 according to a set of X and Y coordinates within the patient room as mapped within hub computer 150 or some other computer such as server 106.

In still other embodiments, zone 116 around the patient support apparatus 110 is defined as any area within the corresponding patient room that is beyond a threshold distance from a doorway of the first patient room. An illustrative doorway is shown to the left in Fig. 1 and a threshold distance may defined about midway between the wall including the doorway and an oppositely facing wall of the patient room. By requiring the caregivers 114a, 114b to be situated within zone 116 adjacent to patient support apparatus 110 for a predetermined period of time, such as about 20 seconds to about 2 minutes just to give a couple arbitrary examples, the likelihood that the outgoing caregiver 114b will communicate relevant information about the patient 112 assigned to patient bed 110 to the incoming caregiver 114a is enhanced.

In some embodiments, one of badges 102 may also be worn by patient 112. In such embodiments, zone 116 may be defined with respect to the badge 102 worn by the patient rather than tag 102c that is attached to patient support apparatus 110. That is, the caregivers 114a, 114b are both required to be in proximity with each of the patients 112 being handed off between the caregivers 114a, 114b by a threshold distance in order for a successful caregiver shift change being considered to occur. In such embodiments, therefore, patient handoffs are able to occur outside of patient rooms such as if the patient is in a treatment room, imaging room, operating room, or the like.

In connection with determining successful shift changes, server 106 further determines whether one of the two identified caregivers 114a, 114b is the correct outgoing caregiver 114b while the other caregiver is the correct incoming caregiver 114a based on the tag data to ensure that the identified caregivers 114a, 114b are effecting a handoff of the correct patient 112 assigned to the patient room or patient support apparatus 110. Of course, as noted above for some embodiments, server 106 also determines whether both caregivers 114a, 114b are in close proximity to the patient support apparatus 110 for a predefined time period. In some embodiments, in addition to determining that both caregivers 114a, 114b are within a first predefined distance from the patient support apparatus 110, server 106 also determines that the caregivers 114a, 114b are within a second predefined distance from each other. For example, an incoming caregiver 114a may still be within the first predefined distance from the patient support apparatus 110 but not within the second predefined distance from an outgoing caregiver 114b if the incoming caregiver 114a is standing in zone 116 at an opposite portion (e.g., diagonally in the case of a rectangle or diametrically across in the case of a circle) of zone 116. As such, a determination of whether tags 102a, 102b of both caregivers 114 and tag 102c of the patient support apparatus 110 are in the respective predefined distances relative to one another provides safeguard against false indication of a successful handoff of the respective patient 112.

In some embodiments, server 106 receives patient data from the patient support apparatus 110 via a communication interface 202 of the patient support apparatus 110 as shown diagrammatically in Fig. 2. The patient data may indicate whether the patient 112 is currently supported on the patient support apparatus 110. As discussed above, the patient support apparatus 110 may determine a presence of the patient 112 on the patient support apparatus 110. For example, the patient support apparatus 110 may determine an amount of weight supported on the patient support apparatus using a scale system 224 integrated into the patient support apparatus 110. If the determined weight does not exceed a predefined weight, the patient support apparatus 110 determines the patient is not supported on the patient support apparatus 110. If, however, the determined weight exceeds the predefined weight, the patient support apparatus 110 determines that the patient 112 is supported on the patient support apparatus 110. This allows the server 106 to affirm that the incoming and outgoing caregivers 114a and 114b are attending to a patient 112 supported on a patient support apparatus 110 and not just an empty patient support apparatus 110. As such, the server 106 may further ensure that the handoff of patient 112 in connection with determining a successful caregiver shift change has been successfully completed only when the patient 112 is present on the patient support apparatus 110 as sensed by a sensor such as one or more load cells of the scale system 224.

Referring once again to Fig. 1, patient support apparatus 110 has a bed frame 124 which includes a base frame 126 with casters 128 and an upper frame or patient support platform 120. The patient support apparatus 110 further includes a headboard 130 at a head end 132, a footboard 134 at a foot end 136, and siderails 138 coupled to the patient support platform 120. A surface or mattress 122 is supported on the patient support platform 120 and, in some embodiments, includes a plurality of inflatable support bladders as is well known in the art. Mattress 122 has an upper surface 140 on which a patient 112 lies. Additionally, the patient support platform 120 includes a number of mattress support sections that support the mattress 122. The mattress support sections include a head section 212, a seat section 214, a thigh section 216, and a foot section 218 as shown diagrammatically in Fig. 2. The head section 212, the thigh section 216, and the foot section 218 are movable relative to the seat section 214 which, in some embodiments, is affixed to upper frame members of the patient support platform 120. For example, the head section 212 may be pivotally raised and lowered relative to the seat section 214, the thigh section 216 may be pivotally raised and lowered relative to the seat section 214, and the foot section 218 may be pivotally raised and lowered relative to the thigh section 216 and the seat section 214.

As shown diagrammatically in Fig. 2, the patient support apparatus 110 further includes a head motor or actuator 206 coupled to the head section 212 of the patient support apparatus 110, a thigh motor or actuator 208 coupled to the thigh section 214, and a foot motor or actuator 210 coupled to the foot section 218. Each of motors 206, 208, 210 may include, for example, an electric motor of a linear actuator. In the illustrative embodiment, a seat section 214 of the patient support apparatus 110 lacks a motor or actuator because it does not articulate relative to the frame members of platform 120. The head motor 260 is operable to raise and lower the head section 212 relative to seat section 214, the thigh motor 208 is operable to raise and lower the thigh section 216 relative to seat section 214, and the foot motor 210 is operable to raise and lower the foot section 218 relative to thigh section 216 and the seat section 214. In addition, the patient support apparatus 110 may include electronic medical record (EMR) charting capability that permits information or data to be charted into a patient's EMR automatically or via commands entered on the patient support apparatus 110. In some embodiments, server 106 is used to chart information regarding caregiver handoffs of patients during caregiver shift changes into the patient's EMR, either automatically at the conclusion of a handoff or shift change, or in response to user inputs by a caregiver at server 106 or at another computer 150, 152, 154.

As also shown diagrammatically in Fig. 2, the patient support apparatus 110 includes a pneumatic system 220 that controls inflation and deflation of the various air bladders of mattress 122. The pneumatic system 220 is represented in Fig. 2 as a single block but that block 220 is intended to represent one or more air sources (e.g., a fan, a blower, a compressor) and associated valves, manifolds, air passages, air lines or tubes, pressure sensors, and the like, as well as the associated electric circuitry, that are typically included in a pneumatic system 220 for inflating and deflating air bladders of mattresses of patient support apparatuses. It should be understood that the inflatable bladders are grouped into various zones of mattress 122. For example, head, seat, thigh and foot zones of mattress 122 each may have one or more bladders located above the respective sections 212, 214, 216, 218 of the same names, just to give one example of a mattress having a plurality of inflatable zones.

The illustrative patient support apparatus 110 includes one or more elevation system motors or actuators 222 to raise, lower, and tilt the patient support platform 120 relative to a base frame 126, which in some embodiments, comprise linear actuators with electric motors. Thus, actuators 222 are sometimes referred to herein as motors 222. The patient support apparatus 110 further includes scale system 224, as mentioned above, to determine a weight of the patient supported on the patient support apparatus 110.

The illustrative patient support apparatus 110 of Fig. 1 includes two user input devices: a caregiver input, which is referred to herein as a main input device 226, and a patient input device 228. The user input devices 226, 228 are electronically coupled to a controller 204 of patient support apparatus 110. For example, the controller 204 may include, among other components customarily included in such devices, a microprocessor 232 and a memory device 230. The memory device 232 may be, for example, a programmable read-only memory device ("PROM") including erasable PROM's (EPROM's or EEPROM's). In use, the memory device 230 is capable of storing, amongst other things, instructions in the form of, for example, a software routine (or routines) which, when executed by the microprocessor, allow the controller 204 to control operation of the features of the patient support apparatus 110.

The user input devices 226, 228 are capable of receiving inputs from a user (e.g., a patient, hospital staff, caregiver, etc.) and, in those embodiments, in which input devices 226, 228 are inputs on a graphical display, are also capable of providing output to the user related to various sensor and/or configuration data of the patient support apparatus 110. Sensor data may include various sensor readings related to current positions, levels, temperatures, pressure levels, etc. of various components of the patient support apparatus 110. In some embodiments, the configuration data may include a designated pressure level of each zone of the plurality of zones of the mattress 122, various settings for positioning the components of the patient support apparatus 110 (e.g., a designated angle of the head section 212 of the patient support apparatus 110 relative to the seat section 214 or relative to horizontal), notifications based on detected events corresponding to the sensor data, and/or any other configurable data that may be set by the user and managed by the controller 204.

Optionally, patient support apparatus 110 includes a proximity sensor 234 as shown diagrammatically in Fig. 2 (in dotted line). Proximity sensor 234 is configured to communicate with tags 102 such as tags 102a, 102b worn by respective caregivers 114a, 114b when the caregivers are within a threshold distance of proximity sensor 234. Thus, in some embodiments, zone 116 is defined by the reception range between tags 102a, 102b and sensor 234. Data indicating that sensor 234 is in wireless communication with one or more tags 102 is among the bed data transmitted from communication interface 202 to one or more of servers 106, 154 and/or computers 150, 152. In some embodiments, control circuitry 204 of the patient bed 110 includes UWB circuitry that is configured to process the wireless signals between proximity sensor 234 and any tags 102 in wireless communication with proximity sensor 234. In this regard, proximity sensor 234 and the UWB circuitry of patient bed 110 operate in a similar manner as transceivers 104 of the high-accuracy locating system.

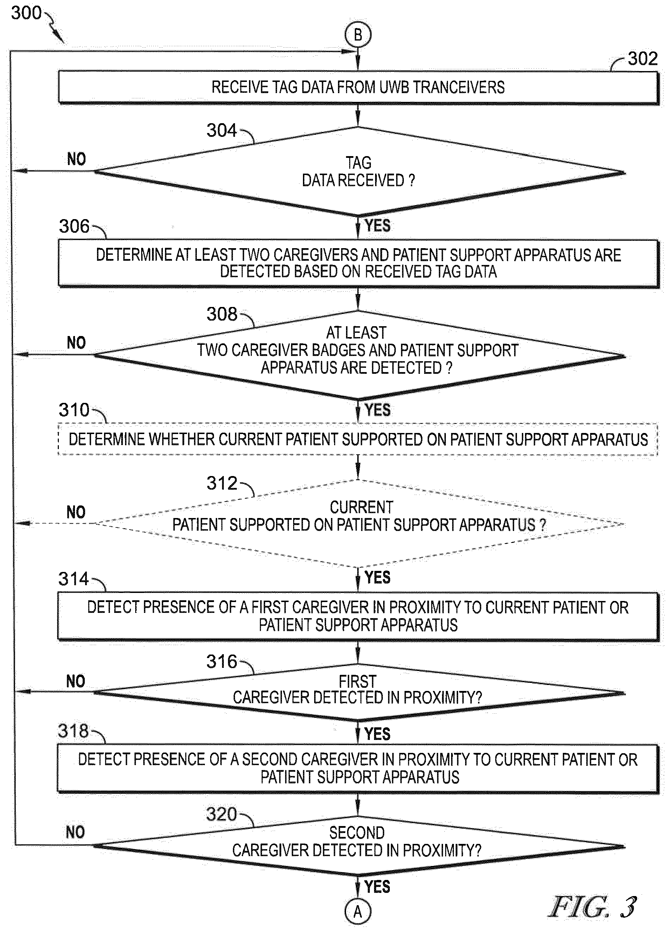

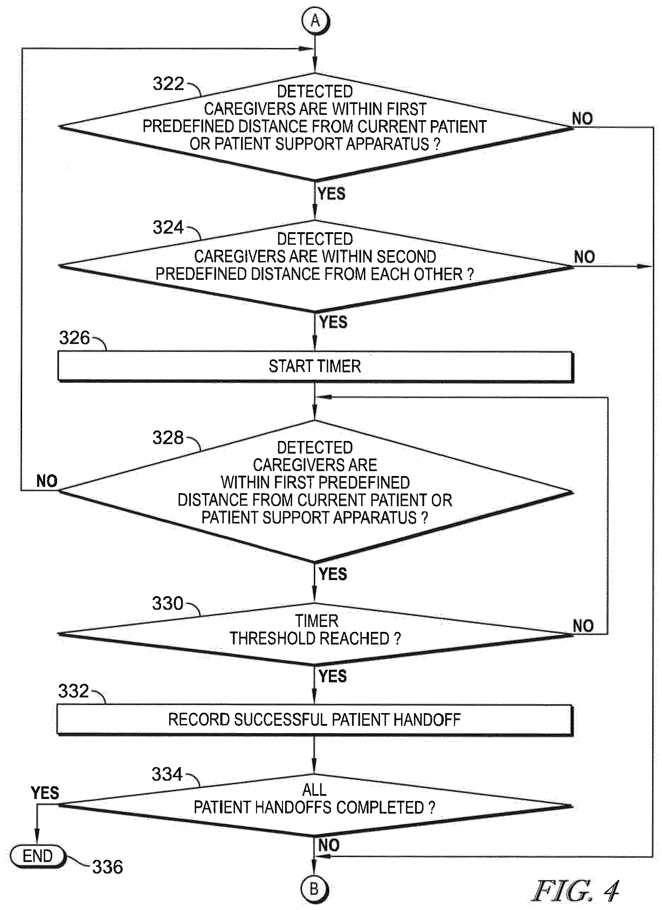

Referring now to Figs. 3 and 4, in use, server 106 executes software to implement a method 300 for receiving data from one or more transceivers 104 to determine whether handoffs of all patients from an outgoing caregiver 114b to one or more incoming caregivers 114a have been completed during a caregiver shift change. According to the method 300, at block 302, the server 106 receives tag data of each tag 102 and transceiver data from each transceiver 104 and proceeds to block 304 to confirm successful receipt of the data from the transceivers 104. As discussed above, the server 106 correlates each transceiver 104 to a location in the healthcare facility based on the transceiver data and selects the tag data of the tags 102 that are in close proximity to each transceiver 104 based on the signal strength. As such, server 106 is configured to selectively analyze the tag data to determine the locations of caregivers 114a, 114b and a patient 112 or a patient support apparatus 110 that are in relative proximity to one another to begin determining a successful handoff. For example, the tag data of a badge 102a or 102b indicates the location and identify of a caregiver 114a, 114b associated with the badge 102a or 102b. The tag data of a tag 102c attached to the patient support apparatus 110 indicates the location and the identification of the patient support apparatus 110. In some embodiments, server 106 includes a database that associates a room number and/or an identity of a patient to be supported on the identified patient support apparatus 110.

If the server 106 has not successfully received tag data and transceiver data at block 304, the method 300 loops back to block 302 to continue to receive tag and transceiver data from one or more transceivers 104. If, however, the server 106 receives the tag and transceiver data at block 304, the method 300 advances to block 306. At block 306, server 106 determines whether at least two caregivers 114a, 114b and patient support apparatus 110 have been detected based on the received tag and transceiver data. In some embodiments, if the tag and transceiver data provides locations of at least two caregivers 114a, 114b, the server 106 further determines whether one of the caregivers is the outgoing caregiver 114b (i.e., end of shift) and the other caregiver is the incoming caregiver 114a (i.e., beginning of shift) based on the identities of the caregivers and their shift schedules.

If server 106 determines that two caregivers 114a, 114b and patient support apparatus 110 are not detected at block 308, server 106 determines that a handoff cannot be achieved, and the method 300 loops back to block 302 to continue receiving new data from transceivers 104 via respective hub computers 150. If, however, the server 106 determines that at least two caregivers 114a, 114b and patient support apparatus 110 are detected, the method 300 advances to block 310 in some embodiments. At block 310, in such embodiments, the patient support apparatus 110 determines whether a current patient is supported on the detected patient support apparatus 110. In some such embodiments, as described above, the patient support apparatus 110 includes an integrated weigh scale system 224 that uses one or more sensors to determine a weight of the patient 112 supported on the patient support apparatus 110. If the measured weight exceeds a threshold level, the patient support apparatus 110 determines that the current patient 112 is supported on the patient support apparatus 110. If the server 106 determines that the patient 112 is not supported on the patient support apparatus 110 at block 312 based on the bed data from the patient support apparatus 110, the method 300 loops back to block 302 to continue receiving new data from the transceivers 104. If, however, server 106 determines that the patient 112 is supported on the patient support apparatus 110 based on the bed data, the method 300 advances to block 314. In other embodiments, algorithm blocks 310, 312 are omitted from the method 300 and the method proceeds from block 308 to block 314 according to the affirmative or yes branch from block 308.

At block 314, the server 106 determines if any one of the detected caregivers 114a, 114b is in proximity by a threshold distance (e.g., within zone 116) of the patient 112 or the patient support apparatus 110, depending upon on the particular embodiment of method 300. If the server 106 determines that one of caregivers 114a, 114b (e.g., caregiver 114a) is in proximity to the patient 112 or patient support apparatus 110 by the threshold distance, the method 300 advances to block 318 to detect the presence of a second one of caregivers 114a, 114b (e.g., caregiver 114b) in proximity to the patient 112 or patient support apparatus 110 by the threshold distance (e.g., also within zone 116). If the server 106 determines that either of the detected caregivers 114a, 114b are not in proximity to the patient 112 or patient support apparatus 110, either at block 318 for the first caregiver or block 320 for the second caregiver, the method 300 loops back to block 302 and proceeds from block 302 as described above. If, however, the server 106 determines at block 320 that there is a second caregiver (e.g., caregiver 114b) who is in proximity to the patient 112 or patient support apparatus 110 along with the first caregiver (e.g., caregiver 114a), the method 300 advances to block 322 of Fig. 4.

At block 322, the server 106 confirms that both caregivers 114a, 114b are still detected in proximity to the patient 112 or patient support apparatus 110. If the server 106 determines that the detected caregivers 114a, 114b are not within the first predefined distance from the patient support apparatus 110 or patient 112 (e.g., not within zone 116) at block 322, the method 300 loops back to block 302 and proceeds from block 302. If, however, the server 106 confirms that two detected caregivers 114a, 114b are within zone 116 having the first predefined distance from the patient 112 or patient support apparatus 110, the server 106 selects those two caregivers 114a, 114b for further monitoring. The confirmation by server at block 322 is somewhat redundant to the determinations by server 106 at blocks 314-320 and so, in some embodiments, block 322 is omitted from method 300.

At block 324, in some embodiments the server 106 further determines whether the selected caregivers 114a, 114b are within a second predefined distance from each other. This is to ensure that that both the selected caregivers 114a, 114b are close enough to the same patient 112 within zone 116 for a successful handoff of the patient 112. In the illustrative example of Fig. 4, if the server 106 determines that the selected caregivers 114a, 14b are not within the second predefined distance from each other at block 324, the method 300 loops back to block 302 and proceeds from block 302. If, however, the server 106 determines at block 324 that the selected caregivers 114a, 114b are within the second predefined distance from each other, the method 300 advances to block 326 and starts a timer. In other embodiments, block 324 is omitted from method 300 such that a determination by server 106 that caregivers 114a, 114b are both in zone 116 (e.g., as determined at blocks 314-320) suffices as part of determining a successful caregiver shift change for the corresponding patient. That is, as long as both caregivers 114a, 114b are anywhere within zone 116, a successful handoff is possible. In some such embodiments in which block 324 is omitted from method 300, the timer is still started as indicated at block 326.

At block 328, server 106 determines whether the selected caregivers 114a, 114b are within the first predefined distance from the patient 112 or patient support apparatus 110 again to ensure that the caregivers 114a, 114b have not moved away from the patient 112 or patient support apparatus 110 during the operation of the timer. If the server 106 determines that the selected caregivers 114a, 114b are no longer within the first predefined distance from the patient support apparatus 110 at block 328, the method 300 loops back to block 322 to determine if there is another set of caregivers 114a, 114b that are within the first predefined distance from the patient 112 or patient support apparatus 110 and, in some embodiments, within the second predefined distance relative to each other. If, however, server 106 determines at block 328 that the selected caregivers 114a, 114b are still within the first predefined distance from the patient support apparatus 110, the method 300 advances to block 330 to determine whether the timer has reached a predefined timer threshold corresponding to the period of time that caregivers 114a, 114b are required to be within proximity of the patient support apparatus 110 or the patient 112 in some embodiments. If the timer has not reached the predefined timer threshold, the method 300 loops back to block 328 to continue determining whether the selected caregivers 114a, 114b are still within the first predefined distance from the patient support apparatus 110 or patient 112 until the timer reaches the predefined timer threshold.

After the timer reaches the predefined timer threshold as determined at block 330, the method 300 advances to block 332 in which the server 106 determines that a caregiver handoff of the current patient was successful and records the successful patient handoff. Subsequently, at block 334, server 106 determines whether all patient handoffs of the outgoing caregiver 114b have been completed. For example, the server 106 compares a list of patients of the outgoing caregivers 114b and checks for handoffs of all of the outgoing caregivers' patients. If server 106 determines that the patient handoffs have not been completed, the method loops back to block 302 to continue receiving new tag and transceiver data to determine respective successful handoffs of other patients of the outgoing caregiver 114b. If, however, server 106 determines that the patient handoffs of all of the patients of all outgoing caregivers 114b have been completed, the method 300 ends as indicated at block 336.

According to the present disclosure, server 106 provides notifications to one or both caregivers 114a, 114b regarding any unsuccessful patient handoffs and/or shift changes. For example, in some embodiments, caregivers 114a, 114b carry wireless communication devices such as pagers, smart phones, personal digital assistants (PDA's), telephone handsets, or the like. Thus, if caregivers 114a, 114b are determined by server not to be within zone 116 or otherwise within proper proximity to each other and/or patient support apparatus 110 and/or the patient 112 in any particular room, server 106 initiates a message to be sent to the wireless communication device(s) of either or both of caregivers 114a, 114b to notify the caregiver 114a, 114b of the problem. The message may be sent via a communication server, for example, which is among the other servers 154 of system 100 in some embodiments. After receipt of the message from server 106 regarding an unsuccessful handoff or shift change, the caregivers 114a, 114b are able to rectify the situation by returning to the appropriate patient room and remaining in zone 116 within the proper proximity with each other and/or with the patient support apparatus 110 or the patient 112 for the threshold period of time, presumably while also exchanging relevant information about the patient being handed off from caregiver 114b to caregiver 114a during the shift change.

Although certain illustrative embodiments have been described in detail above, variations and modifications exist.

Embodiments of the invention can be described with reference to the following numbered clauses, with preferred features laid out in the dependent clauses:
1. A caregiver shift change system for use in a healthcare facility having a plurality of patient rooms, the caregiver shift change system comprising: a patient support apparatus in a first patient room of the plurality of patient rooms; a high-accuracy locating system including a plurality of locating tags, a first tag of the plurality of tags being coupled to a first caregiver, a second tag of the plurality of tags being coupled to a second caregiver, the high-accuracy locating system further having a plurality of transceivers mounted within the healthcare facility and configured to receive tag data from the plurality of tags, and at least one computer coupled to the plurality of transceivers to receive the tag data therefrom and process the tag data to determine a location of each tag of the plurality of tags within the healthcare facility; and a server configured to determine whether a successful caregiver shift change has occurred based on locations of the first and second tags being, for a predetermined period of time, within a threshold proximity of each other and within a zone defined around the patient support apparatus in the first patient room.
2. The caregiver shift change system of clause 1, wherein the high-accuracy locating system operates according to an ultra-wideband (UWB) technology.
3. The caregiver shift change system of either clause 1 or clause 2, wherein the at least one computer uses two way ranging and time difference of arrival data (TDOA) techniques to determine the location of each tag of the plurality of tags.
4. The caregiver shift change system of any preceding clause, wherein the at least one computer uses the tag data from only a subset of the plurality of transceivers to determine the location of each tag of the plurality of tags.
5. The caregiver shift change system of clause 4, wherein the subset is determined based on signal strength of signals that include the tag data and that are communicated between each tag of the plurality of tags and one or more transceivers of the plurality of transceivers.
6. The caregiver shift chance system of clause 5, wherein the subset comprises at least three transceivers from the plurality of transceivers having highest signal strength values as compared to others of the plurality of transceivers.
7. The caregiver shift change system of any preceding clause, wherein the threshold proximity is about three feet.
8. The caregiver shift change system of any one of clauses 1 to 7, wherein the zone defined around the patient support apparatus is defined as an area within about three feet of the patient support apparatus.
9. The caregiver shift change system of any one of clauses 1 to 7, wherein the zone defined around the patient support apparatus is defined as an area within about six feet of a third tag of the plurality of tags mounted to the patient support apparatus.
10. The caregiver shift change system of any one of clauses 1 to 7, wherein the zone defined around the patient support apparatus is defined by a set of X and Y coordinates within the first patient room as mapped within the at least one computer.
11. The caregiver shift change system of any one of clauses 1 to 7, wherein the zone defined around the patient support apparatus is defined as any area within the first patient room that is beyond a threshold distance from a doorway of the first patient room.
12. The caregiver shift change system of any preceding clause, wherein the patient support apparatus includes a sensor that senses a presence of a patient on the patient support apparatus and the server is configured to determine whether a successful caregiver shift change has occurred only when the patient is present on the patient support apparatus as sensed by the sensor.
13. The caregiver shift change system of clause 12, wherein the patient support apparatus includes communication circuitry configured to transmit sensor data from the sensor for receipt by the server.
14. The caregiver shift change system of clause 12, wherein the sensor comprises a weight sensor of a weigh scale system of the patient support apparatus.
15. A method of detecting a successful caregiver shift change in a healthcare facility having patient rooms, the method comprising: receiving, at a computer of a high-accuracy locating system, tag data from a plurality of tags of the high-accuracy locating system, a first tag of the plurality of tags being coupled to a first caregiver, a second tag of the plurality of tags being coupled to a second caregiver, the high-accuracy locating system further having a plurality of transceivers mounted within the healthcare facility and configured to receive tag data from the plurality of tags, and the computer coupled to the plurality of transceivers to receive the tag data therefrom; determining, at the computer, a location of the each tag of the plurality of tags; and determining, at the computer, whether a successful caregiver shift change has occurred based on locations of the first and second tags being, for a predetermined period of time, within a threshold proximity of each other and within a zone defined in a first patient room adjacent a patient support apparatus.
16. The method of clause 15, wherein determining, at the computer, the location of each tag comprises determining the location of the each tag using an ultra-wideband (UWB) technology.
17. The method of either clause 15 or clause 16, wherein determining, at the computer, the location of each tag includes using two way ranging and time difference of arrival (TDOA) techniques.
18. The method of any one of clauses 15 to 17, wherein determining, at the computer, the location of each tag includes using the tag data from only a subset of the plurality of transceivers to determine the location of each tag of the plurality of tags.
19. The method of clause 18, wherein the subset is determined based on signal strength of signals that include the tag data and that are communicated between each tag of the plurality of tags and one or more transceivers of the plurality of transceivers.
20. The method of clause 19, wherein the subset comprises at least three transceivers from the plurality of transceivers having highest signal strength values as compared to others of the plurality of transceivers.
21. The method of any one of clauses 15 to 20, wherein the threshold proximity is about three feet.
22. The method of any one of clauses 15 to 21, wherein the zone defined adjacent the patient support apparatus is defined as an area within about three feet of the patient support apparatus.
23. The method of any one of clauses 15 to 21, wherein the zone defined adjacent the patient support apparatus is defined as an area within about six feet of a third tag of the plurality of tags mounted to the patient support apparatus.
24. The method of any one of clauses 15 to 21, wherein the zone defined adjacent the patient support apparatus is defined by a set of X and Y coordinates within the first patient room as mapped within the computer.
25. The method of any one of clauses 15 to 21, wherein the zone defined adjacent the patient support apparatus is defined as any area within the first patient room that is beyond a threshold distance from a doorway of the first patient room.
26. The method of any one of clauses 15 to 25, wherein the patient support apparatus includes a sensor that senses a presence of a patient on the patient support apparatus and wherein determining, at the computer, whether a successful caregiver shift change has occurred is undertaken only when the patient is present on the patient support apparatus as sensed by the sensor.
27. The method of clause 26, wherein the patient support apparatus includes communication circuitry configured to transmit sensor data from the sensor for receipt by the computer.
28. The method of clause 26, wherein the sensor comprises a weight sensor of a weigh scale system of the patient support apparatus.
29. A caregiver shift change system for use in a healthcare facility having a plurality of patient rooms, the caregiver shift change system comprising: a high-accuracy locating system including a plurality of locating tags, a first tag of the plurality of tags being coupled to a first caregiver, a second tag of the plurality of tags being coupled to a second caregiver, and a third tag of the plurality of tags being coupled to a patient, the high-accuracy locating system further having a plurality of transceivers mounted within the healthcare facility and configured to receive tag data from the plurality of tags, and at least one computer coupled to the plurality of transceivers to receive the tag data therefrom and process the tag data to determine a location of each tag of the plurality of tags within the healthcare facility; and a server configured to determine whether a successful caregiver shift change has occurred based on locations of the first and second tags being, for a predetermined period of time, within a threshold proximity of each other and within a zone defined around the third tag in a patient room of the healthcare facility.
30. The caregiver shift change system of clause 29, wherein the high-accuracy locating system operates according to an ultra-wideband (UWB) technology.
31. The caregiver shift change system of either clause 29 or clause 30, wherein the at least one computer uses two way ranging and time difference of arrival data (TDOA) techniques to determine the location of each tag of the plurality of tags.
32. The caregiver shift change system of any one of clauses 29 to 31, wherein the at least one computer uses the tag data from only a subset of the plurality of transceivers to determine the location of each tag of the plurality of tags.
33. The caregiver shift change system of clause 32, wherein the subset is determined based on signal strength of signals that include the tag data and that are communicated between each tag of the plurality of tags and one or more transceivers of the plurality of transceivers.
34. The caregiver shift chance system of clause 33, wherein the subset comprises at least three transceivers from the plurality of transceivers having highest signal strength values as compared to others of the plurality of transceivers.
35. The caregiver shift change system of any one of clauses 29 to 34, wherein the threshold proximity is about three feet.
36. The caregiver shift change system of any one of clauses 29 to 35, wherein the zone defined around the third tag is defined as an area within about three feet of the third tag.
37. The caregiver shift change system of any one of clauses 29 to 35, wherein the zone defined around the third tag is defined as an area within about six feet of the third tag.
38. A method of detecting a successful caregiver shift change in a healthcare facility having patient rooms, the method comprising: receiving, at a computer of a high-accuracy locating system, tag data from a plurality of tags of the high-accuracy locating system, a first tag of the plurality of tags being coupled to a first caregiver, a second tag of the plurality of tags being coupled to a second caregiver, and a third tag of the plurality of tags being coupled to a patient, the high-accuracy locating system further having a plurality of transceivers mounted within the healthcare facility and configured to receive tag data from the plurality of tags, and the computer coupled to the plurality of transceivers to receive the tag data therefrom; determining, at the computer, a location of the each tag of the plurality of tags; and determining, at the computer, whether a successful caregiver shift change has occurred based on locations of the first and second tags being, for a predetermined period of time, within a threshold proximity of each other and within a zone defined in a first patient room around the third tag.
39. The method of clause 38, wherein determining, at the computer, the location of each tag comprises determining the location of the each tag using an ultra-wideband (UWB) technology.
40. The method of either clause 38 or clause 39, wherein determining, at the computer, the location of each tag includes using two way ranging and time difference of arrival (TDOA) techniques.
41. The method of any one of clauses 38 to 40, wherein determining, at the computer, the location of each tag includes using the tag data from only a subset of the plurality of transceivers to determine the location of each tag of the plurality of tags.
42. The method of clause 41, wherein the subset is determined based on signal strength of signals that include the tag data and that are communicated between each tag of the plurality of tags and one or more transceivers of the plurality of transceivers.
43. The method of clause 42, wherein the subset comprises at least three transceivers from the plurality of transceivers having highest signal strength values as compared to others of the plurality of transceivers.
44. The method of any one of clauses 38 to 43, wherein the threshold proximity is about three feet.
45. The method of any one of clauses 38 to 44, wherein the zone defined around the third tag is defined as an area within about three feet of the third tag.
46. The method of any one of clauses 38 to 44, wherein the zone defined around the third tag is defined as an area within about six feet of the third tag.

## Claims

1. A caregiver shift change system for use in a healthcare facility having a plurality of patient rooms, the caregiver shift change system comprising:
a patient support apparatus in a first patient room of the plurality of patient rooms;
a high-accuracy locating system including a plurality of locating tags, a first tag of the plurality of tags being coupled to a first caregiver, a second tag of the plurality of tags being coupled to a second caregiver, the high-accuracy locating system further having a plurality of transceivers mounted within the healthcare facility and configured to receive tag data from the plurality of tags, and at least one computer coupled to the plurality of transceivers to receive the tag data therefrom and process the tag data to determine a location of each tag of the plurality of tags within the healthcare facility; and
a server configured to determine whether a successful caregiver shift change has occurred based on locations of the first and second tags being, for a predetermined period of time, within a threshold proximity of each other and within a zone defined around the patient support apparatus in the first patient room.

2. The caregiver shift change system of claim 1, wherein the high-accuracy locating system operates according to an ultra-wideband (UWB) technology.

3. The caregiver shift change system of either claim 1 or claim 2, wherein the at least one computer uses two way ranging and time difference of arrival data (TDOA) techniques to determine the location of each tag of the plurality of tags.

4. The caregiver shift change system of any preceding claim, wherein the at least one computer uses the tag data from only a subset of the plurality of transceivers to determine the location of each tag of the plurality of tags.

5. The caregiver shift change system of claim 4, wherein the subset is determined based on signal strength of signals that include the tag data and that are communicated between each tag of the plurality of tags and one or more transceivers of the plurality of transceivers.

6. The caregiver shift chance system of claim 5, wherein the subset comprises at least three transceivers from the plurality of transceivers having highest signal strength values as compared to others of the plurality of transceivers.

7. The caregiver shift change system of any preceding claim, wherein the threshold proximity is about three feet.

8. The caregiver shift change system of any preceding claim, wherein the zone defined around the patient support apparatus is defined as an area within about three feet of the patient support apparatus.

9. The caregiver shift change system of any one of claims 1 to 7, wherein the zone defined around the patient support apparatus is defined as an area within about six feet of a third tag of the plurality of tags mounted to the patient support apparatus.

10. The caregiver shift change system of any one of claims 1 to 7, wherein the zone defined around the patient support apparatus is defined by a set of X and Y coordinates within the first patient room as mapped within the at least one computer.

11. The caregiver shift change system of any one of claims 1 to 7, wherein the zone defined around the patient support apparatus is defined as any area within the first patient room that is beyond a threshold distance from a doorway of the first patient room.

12. The caregiver shift change system of any preceding claim, wherein the patient support apparatus includes a sensor that senses a presence of a patient on the patient support apparatus and the server is configured to determine whether a successful caregiver shift change has occurred only when the patient is present on the patient support apparatus as sensed by the sensor.

13. The caregiver shift change system of claim 12, wherein the patient support apparatus includes communication circuitry configured to transmit sensor data from the sensor for receipt by the server.

14. The caregiver shift change system of either claim 12 or claim 13, wherein the sensor comprises a weight sensor of a weigh scale system of the patient support apparatus.
